(19) **Europäisches Patentamt / European Patent Office / Office européen des brevets**

(11) **EP 3 422 941 B1**

(12) **FASCICULE DE BREVET EUROPEEN**

(45) Date de publication et mention de la délivrance du brevet:
**26.07.2023 Bulletin 2023/30**

(21) Numéro de dépôt: **17711721.5**

(22) Date de dépôt: **01.03.2017**

(51) Classification Internationale des Brevets (IPC):
**A61B 5/11** *(2006.01)* **A61B 5/00** *(2006.01)*

(52) Classification Coopérative des Brevets (CPC):
**A61B 5/1107; A61B 5/6823; A61B 5/725**

(86) Numéro de dépôt international:
**PCT/FR2017/050468**

(87) Numéro de publication internationale:
**WO 2017/149250 (08.09.2017 Gazette 2017/36)**

(54) **DISPOSITIF CARDIAQUE**

KARDIALE VORRICHTUNG

CARDIAC DEVICE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **02.03.2016 FR 1651760**

(43) Date de publication de la demande:
**09.01.2019 Bulletin 2019/02**

(73) Titulaire: **INRIA - Institut National de Recherche en Informatique et en Automatique**
**78150 Le Chesnay (FR)**

(72) Inventeurs:
- **CHAPELLE, Dominique**
  **75001 Paris (FR)**
- **IMPERIALE, Sébastien**
  **92120 Montrouge (FR)**
- **LAURIN, Alexandre**
  **75019 Paris (FR)**
- **MOIREAU, Philippe**
  **75020 Paris (FR)**

(74) Mandataire: **Vidon Brevets & Stratégie**
**16B, rue de Jouanet**
**BP 90333**
**35703 Rennes Cedex 7 (FR)**

(56) Documents cités:
**WO-A1-2015/107374     WO-A1-2015/120330**
**US-A- 6 024 705        US-A1- 2011 066 041**

- **M. CARUEL ET AL: "Dimensional reductions of a cardiac model for effective validation and calibration", BIOMECHANICS AND MODELING IN MECHANOBIOLOGY, vol. 13, no. 4, 8 décembre 2013 (2013-12-08), pages 897-914, XP055320070, Berlin/Heidelberg ISSN: 1617-7959, DOI: 10.1007/s10237-013-0544-6**
- **LAURIN ALEXANDRE ET AL: "A 3D model of the thorax for seismocardiography", COMPUTING IN CARDIOLOGY, N/A, 6 September 2015 (2015-09-06), pages 465-468, XP032865187, ISSN: 2325-8861, DOI: 10.1109/CIC.2015.7408687 ISBN: 978-1-4799-0884-4 [retrieved on 2016-02-16]**

**Description**

Dispositif cardiaque

L'invention concerne le domaine de la surveillance cardiaque.

Il est connu de réaliser des ECG (électrocardiogrammes) pour réaliser le suivi médical d'un patient présentant une maladie cardiaque. Certaines maladies cardiaques nécessitent un suivi quotidien, voire continu. Dans ce contexte, les patients peuvent vivre à domicile, mais il leur est nécessaire de réaliser plusieurs fois par jour des ECG.

Les ECG sont des examens non invasifs mais contraignants dans la mesure où ils nécessitent la pose de plusieurs électrodes de manière assez précise et l'utilisation d'un appareil spécifique. Au jour le jour, l'utilisation de ces appareils pose de nombreux problèmes et réduit de manière significative la mobilité des patients, et l'information qui en découle reste incomplète car elle ne dit rien sur l'état mécanique du système (pressions et débits, etc.).

Il existe des examens appelés SCG (sismocardiogrammes) qui représentent les données de l'activité cardiaque mesurée par un accéléromètre. Cet examen est moins contraignant que l'ECG, car il est réalisé en appliquant un accéléromètre sur le thorax d'un patient et en se basant sur les mesures obtenues. De plus, le SCG contient des informations sur la mécanique du système cardiovasculaire, contrairement à l'ECG.

Cependant, l'interprétation directe des informations contenues dans un SCG est très délicate, en dehors du rythme cardiaque lui-même, de par la complexité des phénomènes sous-jacents, et de fait ces mesures sont peu utilisées à l'heure actuelle.

On connaît la demande US 2011/066041 A1 qui décrit la mesure du mouvement/activité, la fréquence cardiaque et la respiration à partir d'un seul capteur porté sur la poitrine. On connaît la demande US 6 024 705 A qui décrit une détection sismique automatisée de l'ischémie myocardique et mesure connexe des paramètres du débit cardiaque. On connaît la demande WO 2015/107374 A1 qui décrit un dispositif de surveillance cardiaque. On connaît la demande WO 2015/120330 A1 qui décrit un système vetimentaire pour la mesure non-invasive continue de signes vitaux. On connaît l'article M. Caruel et Al « Dimensional réductions of a cardiac model for effective validation and calibration », Biomechanics and modeling in mechabiology, vol. 13, no. 4, 8 décembre 2013, pages 897-914, XP055320070, DOI: 10.1007/s10237-013-0544-6 qui décrit la réduction dimensionnelle d'un modèle cardiaque pour une calibration et une validation effective. On connaît enfin l'article Laurin Alexandre et Al, « A 3D model of the thorax for seismocardiography » COMPUTING IN CARDIOLOGY, N/A, 6 septembre 2015, pages 465-468, XP032865187, DOI : 10.1109/CIC.2015.7408687, qui décrit un modèle 3D pour de la séismocardiographie.

L'invention vient améliorer la situation. À cet effet, l'invention propose un dispositif cardiaque selon la revendication 1.

Ce dispositif est particulièrement avantageux car il permet de réaliser un suivi cardiaque à l'aide de matériels très répandus et peu coûteux, sans mesures invasives, et réalisable hors environnement clinique, donnant accès à des indicateurs cardiaques importants sur la mécanique cardio-vasculaire, hors de portée d'un ECG.

Selon diverses variantes, le dispositif peut présenter une ou plusieurs des caractéristiques suivantes :

- le calculateur est agencé pour calculer un indicateur d'activité cardiaque, en appliquant un modèle thoraco-cardio-vasculaire pour calculer une valeur de signal SCG théorique, et en appliquant au •moins une fonction de correction basée sur la différence entre la valeur de signal SCG théorique et la valeur tirée du filtre,
- le calculateur est agencé pour appliquer au moins un filtre de Kalman ou une combinaison d'un filtre de Kalman avec un observateur de Luenberger dans ladite au moins une fonction de correction,
- l'accéléromètre et le calculateur sont reçus dans un unique boîtier du dispositif,
- l'accéléromètre et le calculateur sont reçus dans deux boîtiers distincts du dispositif,
- le dispositif est agencé pour émettre un signal en fonction d'une comparaison entre un indicateur d'activité cardiaque calculé et une valeur seuil, et
- le calculateur est agencé pour recevoir et traiter des grandeurs liées à l'hémodynamique.

L'invention concerne également un procédé de suivi cardiaque, selon la revendication 8.

Selon ce procédé, l'application du modèle thoraco-cardiovasculaire peut comprendre :

- appliquer un modèle thoraco-cardiovasculaire pour calculer une valeur de signal SCG théorique,
- déterminer une différence entre la valeur de signal SCG théorique et la valeur du signal SCG filtré,
- appliquer au moins une fonction de correction basée sur la différence pour déterminer l'indicateur d'activité cardiaque.

D'autres caractéristiques et avantages de l'invention apparaîtront mieux à la lecture de la description qui suit, tirée d'exemples donnés à titre illustratif et non limitatif, tirés des dessins sur lesquels :

- la figure 1 représente un schéma général d'un dispositif selon invention,
- la figure 2 représente un exemple de mise en oeuvre d'une fonction exécutée par le dispositif de la figure 1,
- la figure 3 représente un exemple de mise en oeuvre d'une opération de la figure 2, et
- les figures 4 à 6 représentent des exemples de signaux obtenus par le dispositif de la figure 1, respectivement avant le filtrage, après le filtrage et après un traitement de mise en forme.

Les dessins et la description ci-après contiennent, pour l'essentiel, des éléments de caractère certain. Ils pourront donc non seulement servir à mieux faire comprendre la présente invention, mais aussi contribuer à sa définition, le cas échéant.

La présente description est de nature à faire intervenir des éléments susceptibles de protection par le droit d'auteur et/ou le copyright. Le titulaire des droits n'a pas d'objection à la reproduction à l'identique par quiconque du présent document de brevet ou de sa description, telle qu'elle apparaît dans les dossiers officiels. Pour le reste, il réserve intégralement ses droits.

En outre, la description détaillée est augmentée de l'annexe A, qui donne la formulation de certaines formules mathématiques mises en oeuvre dans le cadre de l'invention. Cette Annexe est mise à part dans un but de clarification, et pour faciliter les renvois. Elle est partie intégrante de la description, et pourra donc non seulement servir à mieux faire comprendre la présente invention, mais aussi contribuer à sa définition, le cas échéant.

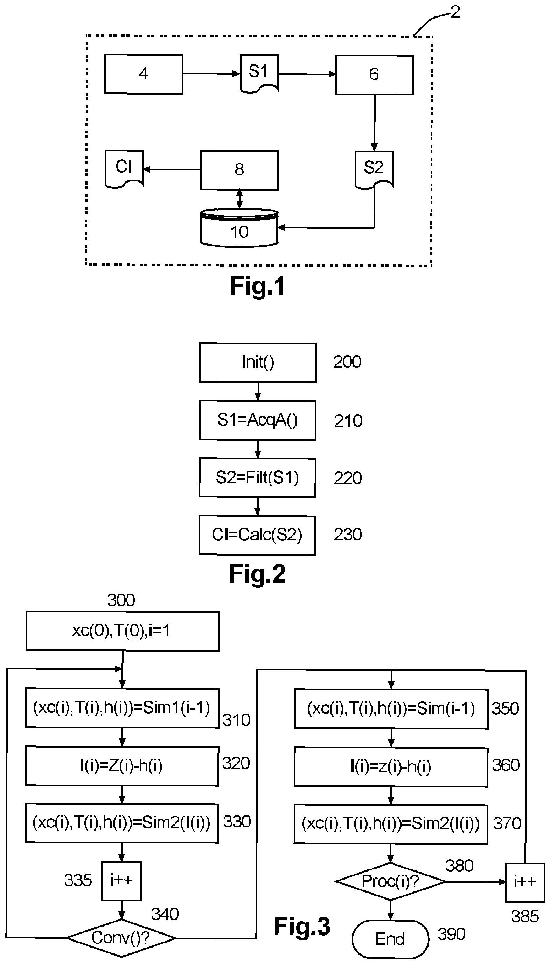

La figure 1 représente un schéma général d'un dispositif 2 selon invention. Le dispositif 2 comprend un accéléromètre 4, un filtre 6, un calculateur 8 et une mémoire 10.

Dans l'exemple décrit ici, le dispositif 2 est un smartphone. Ainsi, le smartphone est disposé contre le thorax d'un patient, de préférence directement au contact de la peau, mais en variante le patient peut garder un T-shirt ou une chemise, un pull risquant d'amortir trop fortement les vibrations produites par les battements du coeur. Ainsi, tous les éléments du dispositif sont contenus dans un seul objet qui, par sa nature, est gardé à proximité par le patient. En variante, les éléments du dispositif 2 pourraient être séparés, l'accéléromètre 4 étant reçu dans un boîtier et maintenu au contact du thorax du patient et communiquant de manière filaire ou sans fil avec un autre boîtier renfermant les autres éléments.

Dans le cadre de l'invention, le filtre 6 et le calculateur 8 sont des éléments accédant directement ou indirectement à la mémoire 10. Ils peuvent être réalisés sous la forme d'un code informatique approprié exécuté sur un ou plusieurs processeurs. Par processeurs, il doit être compris tout processeur adapté. Un tel processeur peut être réalisé de toute manière connue, sous la forme d'un microprocesseur pour ordinateur personnel, d'une puce dédiée de type FPGA ou SoC (« system on chip » en anglais), d'une ressource de calcul sur une grille, d'un microcontrôleur, ou de toute autre forme propre à fournir la puissance de calcul nécessaire à la réalisation décrite plus bas. Un ou plusieurs de ces éléments peuvent également être réalisés sous la forme de circuits électroniques spécialisés tel un ASIC. Une combinaison de processeur et de circuits électroniques peut également être envisagée.

Dans le cadre de l'invention, la mémoire 10 peut être tout type de stockage de données propre à recevoir des données numériques : disque dur, disque dur à mémoire flash (SSD en anglais), mémoire flash sous toute forme, mémoire vive, disque magnétique, stockage distribué localement ou dans le cloud, etc. Les données calculées par le dispositif peuvent être stockées sur tout type de mémoire similaire à la mémoire 10, ou sur celle-ci. Ces données peuvent être effacées après que le dispositif a effectué ses tâches, ou conservées.

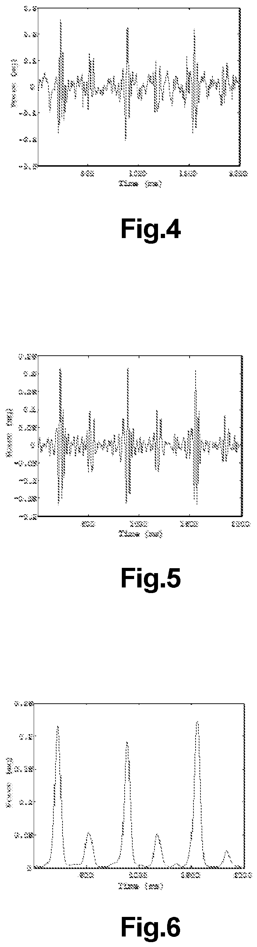

Lorsque le dispositif est placé contre le thorax d'un patient et que l'acquisition de données cardiaques est lancée, l'accéléromètre 4 mesure les vibrations de la cage thoracique du patient et produit un signal S1. La figure 4 représente un exemple d'un signal obtenu en sortie de l'accéléromètre 4.

Le signal S1 est ensuite traité par le filtre 6, notamment afin de débruiter celui-ci. En effet, le signal issu de l'accéléromètre 4 (représenté sur la figure 4) présente une forte composante bruitée, notamment par le fait que l'accéléromètre n'est pas en contact parfait avec le thorax, et que le patient est susceptible de bouger légèrement pendant la mesure, etc.

Ce débruitage vise principalement à filtrer le signal, identifier les sections comportant du bruit important, et y interpoler les données. Le filtre est un filtre passe-bande servant à isoler les fréquences d'intérêt, comme représenté sur la figure 5. On compare ensuite le signal filtré à un signal de référence pour identifier les zones où le bruit empêche l'analyse d'emblée. Les zones propres périphériques aux zones avec bruit sont ensuite utilisées, soit pour analyser les zones avec bruit, soit pour les remplacer.

Le filtre 6 produit un signal S2, dont un exemple est représenté sur la figure 6, qui correspond au signal S1 débruité. Ensuite, le calculateur 8 applique au signal S2 un modèle thoraco-cardiovasculaire afin d'en tirer des informations cardiaques CI. Dans l'exemple décrit ici, le signal S1, le signal S2 et les données CI sont stockés dans la mémoire 10 avec le modèle thoraco-cardiovasculaire.

Le modèle thoraco-cardiovasculaire comprend :

- un modèle cardiovasculaire reliant des paramètres de modélisation du coeur (module d'élasticité, contractilité, tension active maximale des fibres musculaires, etc.), des variables représentatives de l'état du coeur, notamment décrivant ses déformations, et de la circulation sanguine (élastances artérielles, résistances périphériques, pression artérielle et veineuse etc.), une loi représentant les efforts de contact appliqués par le coeur sur la cage thoracique, en fonction des paramètres cardiaques et optionnellement de paramètres représentant la position (verticale / horizontale, etc.) du patient, et

- un modèle thoracique comprenant une fonction de transfert entre les efforts de contact appliqués par le coeur et l'accélération induite dans la cage thoracique.

Dans l'exemple décrit ici, la maladie cardiaque suivie est typiquement une insuffisance cardiaque chronique, par exemple de type « cardiomyopathie dilatée » (CMD). Dans ce cas, le suivi vise à prévenir les risques d'aggravation, en particulier de décompensation aiguë, en adaptant le traitement du patient en fonction de l'évolution de son état.

Dans le cas décrit ici, les grandeurs cardiaques qu'un cardiologue souhaitera surveiller comprennent la variation du rayon de la cavité du ventricule gauche (ci-après y), la déformation des fibres musculaires (ci-après ec), la raideur active cardiaque (ci-après kc), la contrainte active cardiaque (ci-après tc), la pression aortique (ci-après Par) et la pression artérielle distale (ci-après Pd). Ces grandeurs caractéristiques de l'état de fonctionnement cardiaque seront dans la suite rassemblées dans un vecteur xc. D'autres grandeurs d'intérêt concernent certains paramètres de modélisation susceptibles d'évoluer au fil du temps en fonction de l'état du patient, et ces paramètres comprennent notamment la contractilité (ci-après s0), la raideur active (ci-après k0) et la résistance périphérique (ci-après Rd). Ces paramètres sont rassemblés dans un vecteur noté T. Enfin, des indicateurs variés peuvent être tirés du vecteur xc et des paramètres de modélisation T pour permettre de suivre l'évolution de la maladie. Ces indicateurs, comprenant notamment le débit cardiaque (ci-après Q), seront dans la suite désignés par un vecteur CI.

Le vecteur xc et le vecteur T sont reliés par les équations (10) à (60) de l'Annexe A, dans laquelle les équations (10), (20), (30), (40), (50) et (60) désignent les relations indépendantes, et les équations (11) à (18) définissent des membres de l'équation (10). L'article de M. Caruel et al, « Dimensional réductions of a cardiac model for effective validation and calibration », Biomech Model Mechanobiol, 2013 décrit ces équations en détail.

Le système formé par les équations (10) à (60) peut être résumé sous la forme de l'équation (100).

Une fois ce modèle cardiaque posé, un modèle mécanique de force cardio-thoracique est utilisé. Ce modèle comprend une fonction de transfert qui convertit le vecteur xc en force exercée par le coeur sur le thorax.

Ce modèle est résumé sous la forme de l'équation (110), dans laquelle f(t) représente la force exercée par le coeur dans l'état associé au vecteur xc(t), kcon représente une raideur et ycon une distance au repos entre le thorax et le coeur.

À partir de cette force, un modèle thoracique peut être appliqué pour déterminer les déplacements du thorax sous l'effet de la force exercée par le coeur. D'une manière générale, le thorax peut être modélisé comme un ensemble de modes propres, c'est-à-dire des fonctions de déplacement indépendantes les unes des autres, et dépendant de l'application d'une composante de force modale.

Le modèle thoracique peut être résumé sous la forme de l'équation (120), dans laquelle la matrice At représente la dynamique du système de modes propres modélisant le thorax, et xt représente le vecteur décrivant l'état du thorax dans ce modèle. L'équation (125) montre un exemple de mise en oeuvre de ce modèle, dans lequel le vecteur xt est modélisé par at et sa dérivée temporelle, où at est la composante de chaque mode retenu pour le modèle, w est la pulsation propre de chaque mode retenu pour le modèle, xsi est le coefficient d'amortissement de chaque mode retenu pour le modèle, et b est le coefficient d'excitation modale de chaque mode retenu pour le modèle.

Enfin, à partir du vecteur xt décrivant le thorax, il est possible de déterminer une accélération équivalente z(t) du thorax par le biais de l'équation (130), dans laquelle la fonction h() est une fonction de transfert appliquée au vecteur xt de l'état du thorax.

Dans une situation idéale, il serait donc possible de mesurer l'accélération z(t) du thorax pour remonter via les équations (100) à (130) jusqu'au vecteur xc et au vecteur T permettant de suivre l'évolution des paramètres pertinents.

Cependant, le dispositif 2 ne peut pas mesurer de manière parfaite l'accélération du thorax, même après débruitage. Toutes les données qui peuvent être déduites sont donc des approximations.

Afin de pallier ce problème, ainsi que le fait que les opérateurs Ac et At, ainsi que les fonctions f() et h() ne sont pas nécessairement inversibles, la Demanderesse a conçu une fonction Calc() qui vient compléter le modèle décrit plus haut, et a discrétisé ces derniers.

Ainsi, la Demanderesse a introduit avec l'équation (140) un vecteur d'innovation qui mesure la différence entre la mesure débruitée Z(t) et la mesure théorique z(t). Le vecteur d'innovation est ensuite réintroduit dans des fonctions de corrections basées sur un filtre de Kalman, ou sur une combinaison d'un filtre de Kalman avec un observateur de Luenberger comme exposé dans l'article de Moreau et al. « Joint state and parameter estimation for distributed mechanical systems », Comput. Methods Appl. Mech. Engrg. 197 (2008) 659-677. Ces fonctions de correction ont l'avantage d'assurer une convergence dans une large gamme de situations, en quelques boucles de fonctionnement.

En conséquence la Demanderesse a introduit les équations (200) et (220) pour modifier les équations (100) et (120) afin de tenir compte de ces corrections, et introduit l'équation (205) pour tenir compte de leurs effets sur le vecteur T.

Le calculateur 8 peut également recevoir et des signaux mesurés par d'autres capteurs, notamment des mesures non-invasives de grandeurs liées à l'hémodynamique (tensiomètre, tonométrie radiale, photoplethysmogramme, etc.), et les combiner aux données cardiaques déterminées par le calculateur 8 pour en tirer des indicateurs cardiaques permettant le suivi de maladies cardiaques spécifiques.

La figure 2 représente un exemple d'une fonction mise en oeuvre par le dispositif 2. Dans une opération 200, le dispositif 2 exécute une fonction Init(). La fonction Init() initialise le dispositif 2, notamment en sélectionnant des paramètres spécifiques au patient relatifs au modèle cardiovasculaire et/ou au modèle thoracique. Cette fonction peut réaliser une opération de calibration à chaque allumage du dispositif 2, ou réaliser cette calibration de manière périodique ou une fois pour toutes.

Ensuite, dans une opération de 210, l'accéléromètre 4 mesure les vibrations du thorax du patient et en tire le signal S1, puis dans une opération de 220, le filtre 6 exécute une fonction Filt() pour traiter le signal S1 et obtenir le signal S2. Par exemple, la fonction Filt() peut mettre en oeuvre le filtrage décrit dans la thèse de A. Laurin "New timing estimations of cardiovascular events; application to seismocardiography, microneurography, and bloodpressure".

Enfin, dans une opération de 230, le calculateur 8 exécute une fonction Calc() qui applique le modèle thoraco-cardiovasculaire au signal S2 pour en tirer les données cardiaques CI.

La figure 3 représente un exemple de mise en oeuvre de la fonction Calc().

La fonction Calc() comprend deux boucles au fonctionnement identique, dont la première sert à l'initialisation et la deuxième à l'exploitation.

Dans une opération 300, la fonction Calc() débute avec des valeurs d'initialisation des vecteurs xc et T, ainsi qu'avec un indice temporel i.

Ensuite, dans une opération 310, une fonction Sim1() est appliquée avec comme argument l'indice i-1. La fonction Sim1() applique de manière séquentielle une version discrétisée des équations (100) à (130), afin de déterminer quelle serait la valeur de z(t) pour l'instant correspondant à l'instant i, telle que calculée selon le modèle théorique.

Les équations (300) à (330) représentent une possible version discrétisée, par une méthode dite explicite, des équations (100) à (130) appliquées par la fonction Sim1().

Ensuite, dans une opération 320, le calculateur 8 calcule le vecteur d'innovation en appliquant l'équation (140), et la simulation est répétée dans une opération 330 avec une fonction Sim2().

La fonction Sim2() reçoit le vecteur d'innovation I(i) pour appliquer les équations (200) à (220), sous une forme discrétisée de la même manière que les équations (300) à (330), comme cela apparaît avec les équations (400) à (430). Dans la pratique, la fonction Sim2() applique une correction tirée du vecteur d'innovation aux calculs déjà réalisés avec la fonction Sim1(). En variante, la fonction Sim2() pourrait reprendre intégralement les calculs.

Ensuite, dans une opération 335, l'indice i est incrémenté, et, une fonction Conv() est exécutée dans une opération 340 pour comparer la différence entre la valeur h(i) issue de l'opération 330 et la mesure Z(i) issue de l'accéléromètre 4 utilisée à l'opération 320.

Lorsque cette différence est supérieure à un seuil choisi, alors il est considéré que les fonctions de correction ne suffisent pas encore, et la fonction Calc() reprend avec l'opération 310.

Comme mentionné précédemment, la convergence par les fonctions de corrections est atteinte assez rapidement, par exemple à partir des mesures de l'accéléromètre associées à un unique battement cardiaque.

Lorsque la différence est inférieure au seuil choisi, alors il est considéré que les valeurs déterminées sont utiles, et la deuxième boucle commence.

Dans la deuxième boucle, des opérations 350 à 370 identiques aux opérations 310 à 330 sont exécutées afin de déterminer les valeurs courantes du vecteur xt(i) et T(i). Ensuite, dans une opération 380, ces mesures sont affichées et/ou font l'objet d'un traitement relatif au suivi de la maladie avec l'exécution d'une fonction Proc(). La fonction Proc() détermine également s'il reste des données à traiter. Si c'est le cas, alors l'indice i est incrémenté dans une opération 385, et la deuxième boucle reprend avec l'opération 350. Sinon, la fonction Calc() se termine dans une opération 390.

Sur la base des calculs de la fonction Calc() un ensemble de fonctionnalités peuvent être mises en oeuvre, comprenant l'émission d'un signal d'avertissement à l'utilisateur ou à un membre du corps médical (localement ou par une communication à distance), l'affichage des vecteurs xc et T calculés, le calcul d'indicateurs cardiaques CI à partir des vecteurs xc et T, etc.

Le dispositif de l'invention permet de compléter, voire de remplacer plusieurs appareils de mesure qui étaient précédemment utilisés mais non nécessairement synchronisés entre eux. De plus, il permet de produire des mesures directement interprétables dans des situations où le corps médical devait précédemment interpréter des graphes.

La fonction de calcul peut mettre en oeuvre d'autres modèles thoraco-cardiovasculaires que celui donné ici à titre d'exemple, et recevoir et prétraiter des signaux mesurés par d'autres capteurs, et notamment des mesures non-invasives de grandeurs liées à l'hémodynamique (tensiomètre, tonométrie radiale, photoplethysmogramme, etc.).

ANNEXE A

$$\rho d_0 \ddot{y} + \frac{d_0}{R_0}\left(1 + \frac{y}{R_0}\right)\Sigma_{sph} = P_v\left(1 + \frac{y}{R_0}\right)^2 \tag{10}$$

$$\Sigma_{sph} = \sigma_{1D} + 4(1 - C^{-3})\left(\frac{\partial W_e}{\partial J_1} + C\frac{\partial W_e}{\partial J_2}\right) + 2\frac{\partial W_e}{\partial J_4} + 2\eta\dot{C}(1 - 2C^{-6}) \tag{11}$$

$$\sigma_{1D} = E_S \frac{e_{1D} - e_c}{(1 + 2e_c)^2} \tag{12}$$

$$C = \left(1 + \frac{y}{R_0}\right)^2 \tag{13}$$

$$e_{1D} = \frac{C - 1}{2} \tag{14}$$

$$f_{va}(P_v, P_{ar}, P_{ar}) = -4\pi R_0^2 \left(1 + \frac{y}{R_0}\right)^2 \dot{y} \tag{15}$$

$$J_1 = 2C + C^{-2} \tag{16}$$

$$J_4 = C \tag{17}$$

$$W_e(J_1, J_4) = k_1 e^{k_2(J_1 - 3)^2} + k_3 e^{k_4(J_4 - 1)^2} \tag{18}$$

$$(t_c + \mu\dot{e}_c) = E_S \frac{(e_{1D} - e_c)(1 + 2e_{1D})}{(1 + 2e_c)^3} \tag{20}$$

$$\dot{k}_c = -(|\bar{u}| + \alpha|\dot{e}_c|)k_c + n_0 k_0 |\bar{u}| \tag{30}$$

$$\dot{t}_c = -(|\bar{u}| + \alpha|\dot{e}_c|)t_c + n_0 s_0 |\bar{u}| + k_c \dot{e}_c \tag{40}$$

$$C_p \dot{P}_{ar} + \frac{(P_{ar} - P_d)}{R_p} = 4\pi R_0^2 \left(1 + \frac{y}{R_0}\right)^2 \dot{y} \tag{50}$$

$$C_d \dot{P}_d + \frac{(P_d - P_{ar})}{R_p} = \frac{(P_{sv} - P_d)}{R_d} \tag{60}$$

$$\dot{x}_c = A_c(x_c, T, t) \tag{100}$$

6

$$f(t) = k_{con} \, max(y(t) - y_{con}; \, 0) \tag{110}$$

$$\dot{x}_t = A_t(x_t, t) + f(t) \tag{120}$$

$$\begin{bmatrix} \dot{at} \\ \ddot{at} \end{bmatrix} = \begin{bmatrix} 0 & 1 \\ -w^2 & -2 \, xsi \, w \end{bmatrix} \begin{bmatrix} at \\ \dot{at} \end{bmatrix} + \begin{bmatrix} 0 \\ bf(t) \end{bmatrix} \tag{125}$$

$$z(t) = h(\dot{x}_t) \tag{130}$$

$$I(t) = Z(t) - h(\dot{x}_t) \tag{140}$$

$$\dot{x}_c = A_c(x_c, T, t) + K_c(I(t), t) \tag{200}$$

$$\dot{T} = K_T(I(t), t) \tag{205}$$

$$\dot{x}_t = A_t(x_t, t) + f(t) + K_t(I(t), t) \tag{220}$$

$$x_c(t_{i+1}) = A_c(x_c(t_i), T(t_i), t_i)(t_{i+1} - t_i) + x_c(t_i) \tag{300}$$

$$f(t_{i+1}) = k_{con} \, max(y(t_{i+1}) - y_{con}; \, 0) \tag{310}$$

$$x_t(i+1) = (A_t(x_t(t_i), t_i) + f(t_i))(t_{i+1} - t_i) + x_t(t_i) \tag{320}$$

$$z(t_{i+1}) = h\left(\frac{x_t(t_{i+1}) - x_t(t_i)}{(t_{i+1} - t_i)}\right) \tag{330}$$

$$x_c(t_{i+1}) = (A_c(x_c(t_i), T(t_i), t_i) + K_c(I(t_{i+1}), t_{i+1}))(t_{i+1} - t_i) + x_c(t_i) \tag{400}$$

$$T(t_{i+1}) = K_T(I(t_{i+1}), t_{i+1})(t_{i+1} - t_i) + T(t_i) \tag{405}$$

$$f(t_{i+1}) = k_{con} \, max(y(t_{i+1}) - y_{con}; \, 0) \tag{410}$$

$$x_t(i+1) = \left(A_t(x_t(t_i), t_i) + f(t_i) + K_t(I(t_{i+1}), t_{i+1})\right)(t_{i+1} - t_i) + x_t(t_i) \tag{420}$$

$$z(t_{i+1}) = h\left(\frac{x_t(t_{i+1}) - x_t(t_i)}{(t_{i+1} - t_i)}\right) \tag{430}$$

**Revendications**

1. Dispositif cardiaque comprenant un accéléromètre (4) et un filtre (6) agencé pour débruiter un signal sismocardiogramme, SCG, tiré de l'accéléromètre (4), le dispositif cardiaque comprenant en outre un calculateur (8) agencé pour appliquer un modèle thoraco-cardiovasculaire au signal issu du filtre (6) et en tirer au moins un indicateur (CI) d'activité cardiaque, **caractérisé en ce que** le modèle thoraco-cardiovasculaire comprend :

   - un modèle cardiovasculaire reliant des paramètres de modélisation du coeur, des variables représentatives d'un état du coeur et d'une circulation sanguine, et une loi représentant des efforts de contact appliqués par le coeur sur la cage thoracique, et
   - un modèle thoracique comprenant une fonction de transfert entre les efforts de contact appliqués par le coeur et une accélération induite dans la cage thoracique.

2. Dispositif selon la revendication 1, dans lequel le calculateur (8) est agencé pour calculer un indicateur d'activité cardiaque (CI), en appliquant le modèle thoraco-cardiovasculaire pour calculer une valeur de signal SCG théorique, et en appliquant au moins une fonction de correction basée sur la différence entre la valeur de signal SCG théorique et la valeur tirée du filtre (6).

3. Dispositif selon la revendication 2, dans lequel le calculateur (8) est agencé pour appliquer au moins un filtre de Kalman ou une combinaison d'un filtre de Kalman avec un observateur de Luenberger dans ladite au moins une fonction de correction.

4. Dispositif selon l'une des revendications précédentes, dans lequel l'accéléromètre (4) et le calculateur (8) sont reçus dans un unique boîtier du dispositif (2).

5. Dispositif selon l'une des revendications 1 à 3, dans lequel l'accéléromètre (4) et le calculateur (8) sont reçus dans deux boîtiers distincts du dispositif (2).

6. Dispositif selon l'une des revendications précédentes, agencé en outre pour émettre un signal en fonction d'une comparaison entre l'indicateur d' activité cardiaque calculé et une valeur seuil.

7. Dispositif selon l'une des revendications précédentes, dans lequel le calculateur (8) est agencé pour recevoir et traiter des grandeurs liées à l'hémodynamique.

8. Procédé de suivi cardiaque, comprenant :

   - obtenir (210) un signal sismocardiogramme, SCG, d'un accéléromètre,
   - filtrer (220) le signal SCG,
   - appliquer (230) un modèle thoraco-cardiovasculaire au signal SCG filtré et en tirer au moins un indicateur d'activité cardiaque, **caractérisé en ce que** le modèle thoraco-cardiovasculaire comprend :

      - un modèle cardiovasculaire reliant des paramètres de modélisation du coeur, des variables représentatives d'un état du coeur et d'une circulation sanguine, et une loi représentant des efforts de contact appliqués par le coeur sur la cage thoracique, et
      - un modèle thoracique comprenant une fonction de transfert entre les efforts de contact appliqués par le coeur et une accélération induite dans la cage thoracique.

9. Procédé selon la revendication 8 dans lequel l'application du modèle thoraco-cardiovasculaire comprend :

   - appliquer le modèle thoraco-cardiovasculaire pour calculer une valeur de signal SCG théorique,
   - déterminer une différence entre la valeur de signal SCG théorique et la valeur du signal SCG filtré,
   - appliquer au moins une fonction de correction basée sur la différence pour déterminer l'indicateur d'activité cardiaque.

**Patentansprüche**

1. Kardiale Vorrichtung, umfassend einen Beschleunigungsmesser (4) und einen Filter (6), der dazu eingerichtet ist, ein seismokardiografisches (SCG) Signal zu entstören, das dem Beschleunigungsmesser (4) gewonnen wird, wobei die kardiale Vorrichtung außerdem einen Rechner (8) umfasst, der dazu eingerichtet ist, ein Thorax-Herz-Gefäß-Modell auf das vom Filter (6) ausgegebene Signal anzuwenden und daraus mindestens einen Indikator (CI) für Herzaktivität zu gewinnen, **dadurch gekennzeichnet, dass** das Thorax-Herz-Gefäß-Modell umfasst:

- ein Herz-Gefäß-Modell, das Herzmodellierungsparameter, Variablen, die für einen Zustand des Herzens und eine Blutzirkulation repräsentativ sind, mit einem Gesetz verknüpft, das die vom Herzen auf den Brustkorb aufgebrachten Kontaktkräfte repräsentiert, und
- ein Thoraxmodell, das eine Transferfunktion zwischen den vom Herzen auf den Brustkorb aufgebrachten Kontaktkräften und einer in den Brustkorb induzierten Beschleunigung umfasst.

2. Vorrichtung nach Anspruch 1, wobei der Rechner (8) dazu eingerichtet ist, einen Indikator für die Herzaktivität (CI) zu berechnen, indem das Thorax-Herz-Gefäß-Modell angewandt wird, um einen theoretischen Wert des SGC-Signals zu berechnen, und mindestens eine Korrekturfunktion basierend auf der Differenz zwischen dem theoretischen Wert des SCG-Signals und dem aus dem Filter (6) gewonnenen Wert anzuwenden.

3. Vorrichtung nach Anspruch 2, wobei der Rechner (8) dazu eingerichtet ist, mindestens einen Kalman-Filter oder eine Kombination eines Kalman-Filters mit einem Luenberger-Beobachter in der mindestens einen Korrekturfunktion anzuwenden.

4. Vorrichtung nach einem der vorhergehenden Ansprüche, wobei der Beschleunigungsmesser (4) und der Rechner (8) in einem einzigen Gehäuse der Vorrichtung (2) aufgenommen sind.

5. Vorrichtung nach einem der Ansprüche 1 bis 3, wobei der Beschleunigungsmesser (4) und der Rechner (8) in zwei unterschiedlichen Gehäusen der Vorrichtung (2) aufgenommen sind.

6. Vorrichtung nach einem der vorhergehenden Ansprüche, die außerdem dazu eingerichtet ist, ein Signal in Abhängigkeit von einem Vergleich zwischen dem berechneten Indikator für die Herzaktivität und einem Schwellenwert auszugeben.

7. Vorrichtung nach einem der vorhergehenden Ansprüche, wobei der Rechner (8) dazu eingerichtet ist, mit der Hämodynamik verbundene Größen zu empfangen und zu verarbeiten.

8. Verfahren zur Herzüberwachung, umfassend:

- Erhalten (210) eines seisokardiografischen (SCG) Signals eines Beschleunigungsmessers,
- Filtern (220) des SCG-Signals,
- Anwenden (230) eines Thorax-Herz-Gefäß-Modells auf das gefilterte SCG-Signal und daraus Gewinnen mindestens eines Indikators für die Herzaktivität, **dadurch gekennzeichnet, dass** das Thorax-Herz-Gefäß-Modell umfasst:

- ein Herz-Gefäß-Modell, das Herzmodellierungsparameter, Variablen, die für einen Zustand des Herzens und eine Blutzirkulation repräsentativ sind, mit einem Gesetz verknüpft, das die vom Herzen auf den Brustkorb aufgebrachten Kontaktkräfte repräsentiert, und
- ein Thoraxmodell, das eine Transferfunktion zwischen den vom Herzen auf den Brustkorb aufgebrachten Kontaktkräften und einer in den Brustkorb induzierten Beschleunigung umfasst.

9. Verfahren nach Anspruch 8, wobei die Anwendung des Thorax-Herz-Gefäß-Modells umfasst:

- Anwenden des Thorax-Herz-Gefäß-Modells zum Berechnen eines theoretischen Wertes des SCG-Signals,
- Ermitteln einer Differenz zwischen dem theoretischen Wert des SCG-Signals und dem Wert des gefilterten SCG-Signals,
- Anwenden mindestens einer Korrekturfunktion basierend auf der Differenz zum Ermitteln des Indikators für die Herzaktivität.

**Claims**

1. A cardiac device comprising an accelerometer (4) and a filter (6) arranged to remove noise from a seismocardiogram, SCG, signal derived from the accelerometer (4), the cardiac device further comprising a calculator (8) arranged to apply a thoraco-cardiovascular model to the signal coming from the filter (6) and to derive at least one cardiac activity indicator (CI) therefrom, **characterized in that** the thoraco-cardiovascular model comprises:

   - a cardiovascular model linking parameters for modeling the heart, variables representative of the state of the heart and blood, and a law representing the contact forces applied to the thoracic cavity by the heart, and
   - a thoracic model comprising a transfer function between the contact forces applied by the heart and the acceleration induced in the thoracic cavity.

2. The device according to claim 1, wherein the calculator (8) is arranged to compute a cardiac activity indicator (CI) by applying the thoraco-cardiovascular model in order to compute a theoretical SCG signal value, and by applying at least one correction function based on the difference between the theoretical SCG signal value and the value derived from the filter (6).

3. The device according to claim 2, wherein the calculator (8) is arranged to apply at least one Kalman filter or a combination of a Kalman filter and a Luenberger observer in said at least one correction function.

4. The device according to one of the preceding claims, wherein the accelerometer (4) and the calculator (8) are accommodated in a single housing of the device (2).

5. The device according to one of claims 1 to 3, wherein the accelerometer (4) and the calculator (8) are accommodated in two separate housings of the device (2).

6. The device according to one of the preceding claims, furthermore arranged to transmit a signal as a function of a comparison between the computed cardiac activity indicator and a threshold value.

7. The device according to one of the preceding claims, wherein the calculator (8) is arranged to receive and process variables linked to hemodynamics.

8. A cardiac monitoring method, comprising:

   - obtaining (210) a seismocardiogram, SCG, signal from an accelerometer,
   - filtering (220) the SCG signal,
   - applying (230) a thoraco-cardiovascular model to the filtered SCG signal and taking at least one cardiac activity indicator therefrom, **characterized in that** the thoraco-cardiovascular model comprises:

      - a cardiovascular model linking parameters for modeling the heart, variables representative of the state of the heart and blood, and a law representing the contact forces applied to the thoracic cavity by the heart, and
      - a thoracic model comprising a transfer function between the contact forces applied by the heart and the acceleration induced in the thoracic cavity.

9. The method according to claim 8, wherein the application of the thoraco-cardiovascular model comprises:

   - applying the thoraco-cardiovascular model in order to compute a theoretical SCG signal value,
   - determining a difference between the theoretical SCG signal value and the value of the filtered SCG signal,
   - applying at least one correction function based on the difference in order to determine the cardiac activity indicator.

**Fig.1**

```
          ┌─────────┐        ┌─────┐        ┌─────────┐
          │    4    │───────▶│ S1  │───────▶│    6    │
          └─────────┘        └─────┘        └─────────┘
                                                 │
                                                 ▼
   ┌────┐     ┌─────────┐                   ┌─────┐
   │ CI │◀────│    8    │                   │ S2  │
   └────┘     └─────────┘                   └─────┘
                   ↕                             │
              ┌─────────┐                        │
              │   10    │◀───────────────────────┘
              └─────────┘
```

2

**Fig.2**

| | |
|---|---|
| Init() | 200 |
| S1=AcqA() | 210 |
| S2=Filt(S1) | 220 |
| CI=Calc(S2) | 230 |

**Fig.3**

300

$xc(0), T(0), i=1$

$(xc(i), T(i), h(i)) = Sim1(i-1)$   310

$I(i) = Z(i) - h(i)$   320

$(xc(i), T(i), h(i)) = Sim2(I(i))$   330

335   i++

340   Conv()?

$(xc(i), T(i), h(i)) = Sim(i-1)$   350

$I(i) = z(i) - h(i)$   360

$(xc(i), T(i), h(i)) = Sim2(I(i))$   370

Proc(i)?   380   i++   385

End   390

**Fig.4**

**Fig.5**

**Fig.6**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2011066041 A1
- US 6024705 A
- WO 2015107374 A1
- WO 2015120330 A1

**Littérature non-brevet citée dans la description**

- **M. CARUEL et al.** Dimensional réductions of a cardiac model for effective validation and calibration. *Biomechanics and modeling in mechabiology,* 08 Décembre 2013, vol. 13 (4), 897-914
- **LAURIN ALEXANDRE et al.** A 3D model of the thorax for seismocardiography. *COMPUTING IN CARDIOLOGY, N/A,* 06 Septembre 2015, 465-468
- **M. CARUEL et al.** Dimensional réductions of a cardiac model for effective validation and calibration. *Biomech Model Mechanobiol,* 2013
- **DE MOIREAU et al.** Joint state and parameter estimation for distributed mechanical systems. *Comput. Methods Appl. Mech. Engrg.,* 2008, vol. 197, 659-677
- **A. LAURIN.** *New timing estimations of cardiovascular events; application to seismocardiography, microneurography, and bloodpressure*